# EUROPEAN PATENT APPLICATION

(11) **EP 4 015 630 A1**
(43) Date of publication of application: **22.06.2022**
(21) Application number: 21214708.6
(22) Date of filing: 15.12.2021
(51) Int. Cl.: C12N 15/10, C12Q 1/6806

(54) **NUCLEIC ACID ISOLATION KIT AND METHOD**

(30) Priority: 15.12.2020 CZ 202038366 U
(71) Applicant: Univerzita Palackého v Olomouci, 779 00 Olomouc (CZ)
(72) Inventor: Dzubak, Petr, 75103 Citov (CZ); Hajduch, Marian, 79305 Norbercany (CZ); Gurska, Sona, 02062 Horovce (SK); Koudelakova, Vladimira, 78401 Litovel (CZ); Jaworek, Hana, 77900 Olomouc (CZ)
(74) Representative: Harber IP s.r.o.

(57) **Abstract**

The invention provides a nucleic acid isolation kit, which contains:
- an aqueous lysis buffer containing 0.05 to 0.07 M sodium citrate dihydrate, 10 to 20 µM 3,3',5,5'-tetrabromo-m-cresolsulfophthalein (bromocresol green) and 50 to 60% (w/v) guanidine thiocyanate, and having a pH of 6;
- an aqueous reducing solution containing 10 to 20% (w/v) tris(2-carboxyethyl)phosphine (TCEP);
- paramagnetic beads;
- a washing solution which is 0.5 to 2 mM citrate buffer in a 1:1 to 1:5 mixture of water and ethanol;
- a precipitating component which is ethanol;
- an elution solution which is 3 to 7 mM Tris buffer.

Provided is also a method of using the kit.

## Description

### Field of Art

The invention relates to a kit and a method for isolation of RNA and DNA from samples.

### Background Art

RNA isolation is a procedure for obtaining RNA from samples, for example for biochemical, molecular-biological or forensic analyses and other methods. Current RNA isolation methods are commonly based on extraction with guanidine thiocyanate, phenol and chloroform. The problem with RNA isolation is the presence of RNases, so there is a need to develop methods that are straightforward and minimize contact with tools and surfaces.

It is desirable to be able to isolate the DNA from the samples in parallel in sufficient quality. A procedure allowing a parallel isolation of RNA and DNA would remove the need for splitting the sample into two portions for two separate isolation procedures and would thus reduce the amount of the material which is necessary for nucleic acid isolation, and increase the yield of the isolated nucleic acids.

### Disclosure of the Invention

The present invention provides a nucleic acid isolation kit, which contains:
- an aqueous lysis buffer containing 0.05 to 0.07 M sodium citrate dihydrate, 10 to 20 µM 3,3',5,5'-tetrabromo-m-cresolsulfophthalein (bromocresol green) and 50 to 60% (w/v) guanidine thiocyanate, and having a pH of 6;
- an aqueous reducing solution containing 10 to 20% (w/v) tris(2-carboxyethyl)phosphine (TCEP);
- paramagnetic beads;
- a washing solution which is 0.5 to 2 mM citrate buffer in a 1:1 to 1:5 mixture of water and ethanol;
- a precipitating component which is ethanol;
- an elution solution which is 3 to 7 mM Tris buffer.

The term "nucleic acid" includes DNA and RNA.

Preferably, the lysis buffer contains 0.06 M sodium citrate dihydrate, 14 µM 3,3',5,5'-tetrabromo-m-cresol sulfophthalein, and 56 to 57% (w/v) guanidine thiocyanate.

Preferably, the reducing solution contains 14 to 15% (w/v) tris(2-carboxyethyl)phosphine in water.

Preferably, the washing solution contains 1 mM citrate buffer in a 1:1 to 1:5 mixture of water and ethanol, having pH 6. Preferably, the solvent in the washing solution is 75% (v/v) aqueous ethanol.

Preferably, the elution solution is 5 mM Tris buffer having pH 8.5. Tris buffer is a solution of Tris and Tris HCl. Tris is tris(hydroxymethyl)aminomethane, and Tris HCl is the hydrochloride salt of this base.

The water used in the kit is preferably water free of RNases and DNases.

The paramagnetic beads are commercially available, and are usually commercially available as paramagnetic beads with a nucleic acid-binding surface coating.

The kit preferably further includes a nucleic acid carrier, preferably a nucleic acid carrier based on linear polyacrylamide. These nucleic acid carriers are commercially available. The nucleic acid carriers are RNA carriers and DNA carriers.

In some embodiments, the components: lysis buffer, reducing solution, RNA carrier, paramagnetic beads, and precipitation solution may be mixed into one mixed component of the kit. Preferably, the lysis buffer, the reducing solution, the RNA carrier and the precipitation solution in the mixed component are mixed in volume ratios of 10³ : 5 to 10 : 5 to 10 : 10³ to 3x10³, more preferably in a volume ratio of 10³: 6: 7: 2×10³.

The present invention further provides a method for isolation of nucleic acid from a sample, comprising the steps of
- providing a first mixture containing lysis buffer, reducing solution, paramagnetic beads, and precipitation solution, and optionally nucleic acid carrier,
- mixing the said first mixture with the sample to form a second mixture,
- separating the paramagnetic beads from the liquid phase of the second mixture,
- mixing the paramagnetic beads with a washing solution and then separating the paramagnetic beads from the liquid phase, this step is optionally repeated,
- mixing the paramagnetic beads with an elution solution and separating the nucleic acid-containing supernatant from the paramagnetic beads.

The steps of separating of the paramagnetic beads from the supernatant are preferably carried out by placing the mixture on a magnetic stand or on a magnetic pad, allowing the paramagnetic beads to settle by the action of the magnetic field,

In the method of the invention, the nucleic acid is released from the sample by the action of the first mixture, and binds to paramagnetic beads. Then other components of the original samples are washed away while the nucleic acid is still bound to the paramagnetic beads, and then the nucleic acid is eluted from the paramagnetic beads. The method is simple, avoids unnecessary handling, and the isolated nucleic acid does not need to be repeatedly contacted with different vessels or equipment which would risk its contamination or decomposition. The whole isolation procedure can be carried out in one vessel or multi-well plate.

### Examples of carrying out the Invention

### Example 1: Preparation of the Kit

First, stock solutions are prepared:
- 1M Tris HCl: 7.88 g of Tris base was weighed and made up to 50 ml with water. The buffer was filtered using a 0.22 µm filter in a laminar box. The solution is freezed in 1 ml aliquots and stored at -20 °C ± 3 °C.
- Tris base 1.5M: 9.0855 g of Tris base was weighed and made up to 50 ml with water. The buffer was filtered using a 0.22 µm filter in a laminar box. The solution is freezed in 1 ml aliquots and stored at -20 °C ± 3 C.
- Citric acid 2M: 3.842 g of citric acid was weighed and made up to 10 ml with water. The solution was filtered through a 0.22 µm filter in a laminar box and stored at 4 °C ± 3 °C.
- 0.2M citrate buffer, pH 6: 2.941 g of sodium citrate dihydrate was weighed, dissolved in 6 ml of water and adjusted to pH 6 with 2M citric acid and/or 2M NaOH. The solution was filtered through a 0.22 µm filter in a laminar box and stored at 4 °C ± 3 °C.
- Bromocresol green 14mM: 97.7 mg of bromocresol green was weighed and dissolved in 10 ml of 100% ethanol. The solution was freezed in 1 ml aliquots at -20 °C ± 3 °C.

In the next step, the individual components of the kit were prepared:
- Lysis buffer (LB): 3 ml of a stock solution of 0.2M sodium citrate dihydrate, pH 6, was pipetted into a 50 ml tube, to this solution were added guanidine thiocyanate 5.67 g and 2.5 ml of water. The pH of the obtained solution was adjusted with 2M citric acid solution and optionally 2M sodium hydroxide to the final pH = 6, after dissolving and buffering, the solution was made up to a final volume of 10 ml with water. 10 µl of 14mM solution of bromocresol green was added to the buffer. The buffer was filtered using a 0.22 µm filter in a laminar box. The lysis buffer is stored in a refrigerator, warmed to room temperature before use, and the precipitated crystals are dissolved by stirring with the vial.
- The RNA carrier was "Acryl carrier" purchased from Top-Bio s.r.o., Catalog No. C081. The solution is stored at 4 °C ± 3 °C.
- The reducing solution was prepared by dissolving 1.43 g of TCEP in 10 ml of H₂O. The solution is stored at 4 °C ± 3 °C.
- Paramagnetic beads were obtained commercially, and used as a suspension in 50% (v/v) aqueous isopropanol. Paramagnetic beads are supplied at a concentration of 150 mg/ml in 100% isopropanol. The suspension is stored at 4 °C ± 3 °C.
- The washing solution was prepared by adding 75 mL of 100% ethanol and 24.5 mL of H₂O to 0.5 mL of citrate buffer (0.2M, pH 6). The solution is stored at room temperature.
- The precipitating component is 100% ethanol. The solution is stored at room temperature.
- The elution solution, which is a 5 mM Tris solution at pH 8.5, was prepared by mixing Tris HCl 1M 1.58 mL and Tris Base 1.5M 2.28 mL and diluting with RNA water to 1 L. The solution is stored at 4 °C ± 3 °C.

The water for the preparation of all components was free of RNases and DNases.

Composition of one kit, destined for 96 isolation reactions in one 96-well plate, is as follows:
- Lysis buffer (LB) - 10 ml
- RNA carrier (C) - 75 µl
- Reducing solution (R) - 65 µl
- Paramagnetic beads (MB) - 2,4 ml
- Washing solution (WB) - 1×30 ml, 1×40ml
- Precipitating component (PS) - 30 ml
- Elution solution (EB) - 7,5 ml

### Example 2: Use of the Kit for RNA/DNA isolation

This example describes the use of the kit prepared in Example 1.

The lysis buffer is allowed to warm up to room temperature before use. Any crystals in the solution must be dissolved before use. To 10 ml of the lysis buffer, reducing solution (R) 60 µl, RNA carrier (C) 70 µl, paramagnetic beads (MB) 2 ml and precipitating component (PS) 20 ml are added. This is how the first mixture (for isolation of RNA/DNA) is prepared. Then a part of the sample can be taken as a negative control, or an internal isolation control can be added according to the PCR kit manufacturer's recommendation (for further steps after the isolation step).

320 µl of the first mixture per well is pipetted into a 96-well lysis plate. 100 µl of sample per well is added to the lysis plate and the plate is sealed with foil. Subsequently, the lysis plate is heated at 65 °C for 10 minutes and then cooled to room temperature. The contents of each well are mixed thoroughly and incubated for 5 minutes at room temperature. The plate is transferred to a magnetic pad, where the paramagnetic beads and supernatant are separated after 2 minutes. The supernatant is aspirated from the plate positioned on a magnetic pad.

350 µl per well of the washing solution is added to the samples, stirred, and incubated for 2 minutes before separating the paramagnetic beads and the supernatant. The supernatant is aspirated from the plate positioned on a magnetic pad. 150 µl per well of the washing solution is added to the samples, stirred, and incubated for 2 minutes before separating the paramagnetic beads and the supernatant. The supernatant is aspirated from the plate positioned on a magnetic pad.

The plate is transferred to a tempered mat (45 ° C) and dried for about 15 minutes. It is then transferred off the tempered plate, and 50 µl of the elution solution is added to the wells containing the paramagnetic beads, mixed, and incubated for 5 minutes while warming to 70 °C. The plate is transferred to a magnetic pad, where the paramagnetic beads and the supernatant separate from each other after 2 minutes. An appropriate volume (45 µl) of the supernatant is pipetted into a clean plate. The RNA/DNA thus isolated is ready for immediate use or the plate can be covered, freezed and the samples stored for later use.

### Example 3: Use of the isolated RNA for viral diagnostic.

In this experiment, the possibility of isolation and detection of RNA viruses, particularly SARS-Cov-2 virus were studied. The isolation kit according to Example 1 was used.

The SARS-Cov-2 positive and negative patient samples were selected and isolated. Isolation of RNA was performed using the Zybio Nucleic Acid Extraction Kit T200-96 (Zybio) and by the RNA/DNA isolation kit according to Example 1. RT-PCR kin Novel Coronavirus (2019-nCov) Real Time Multiplex RT-PCR Kit (Detection 3 genes) (Liferiver, Shanghai UJ Bio-Tech Co.) was used for the detection.

Isolation and detection were performed according to the manufacturer's instructions. Tests were also performed with negative and positive controls; controls from the above commercial kit were used.

The cumulative results are shown in Table 1 below. It was shown that the invented isolation kit has comparable sensitivity for the detection of the viral RNA positivity (Sensitivity 96.1%, Specificity 100%, Negative predictive value 97.6%, Positive predictive value 100%, Accuracy 98.5%).

Table 1: Cumulative results of SARS-CoV-2 detection assay in patient samples which were isolated by the two different methods, RNA/DNA isolation kit according the present invention and Zybio Nucleic Acid Extraction Kit T200-96.

| | | Cumulative result | | |
|---|---|---|---|---|
| PCR -SARS-CoV-2 | | Positive | Negative | Total |
| Zybio -Nucleic Acid Extraction Kit | Positive | 125 | 0 | 125 |
| | Negaitve | 3 | 201 | 204 |
| | Total | 128 | 201 | 329 |
| RNA/DNA isolation kit (invention) | Positive | 123 | 9 | 123 |
| | Negaitve | 5 | 201 | 206 |
| | Total | 128 | 201 | 329 |

### Example 4: Use of the isolated RNA with RT-PCR viral detection kit using the internal PCR control based on extracted genomic RNA.

In this experiment, the possibility of isolation and detection of an RNA virus, specifically SARS-Cov-2 virus, were studied together with the method using as the internal positive control detection of the expression of the human GAPDH gene.

SARS-Cov-2 positive and negative patient samples were harvested. Isolation of RNA was performed using the RNA/DNA isolation kit according to Example 1.

SARS-CoV-2 Nucleic Acid Detection Kit (Zybio) was used for the detection. As a part of the detection kit the GAPDH gene detection primers are available and this gene serves as the internal sampling control for each sample.

Detection was performed according to the manufacturer's instructions. Tests were also performed with negative and positive controls; controls from the above commercial kit were used.

The results are shown in Table 2 below.

**Table 2: The results of SARS-CoV-2 detection assay in patient samples which were isolated by the RNA/DNA isolation kit according the present invention and detected by SARS-CoV-2 Nucleic Acid Detection Kit (Zybio) detection. As a part of the detection kit the GAPDH gene serves as the internal sampling control for each sample. ND = not detected**

| | ROX/TEXAX-RRP | CYS-E-gene | Fam - N-gene | HEX/MC - GAPDH gene |
|---|---|---|---|---|
| ID | Cp | Cp | Cp | Cp |
| CoV274908 | ND | ND | ND | 20.67621643 |
| CoV274909 | ND | ND | ND | 23.38331149 |
| CoV274910 | ND | ND | ND | 25.19083777 |
| CoV274911 | ND | ND | ND | 22.52758143 |
| CoV274912 | ND | ND | ND | 28.56964865 |
| CoV274913 | ND | ND | ND | 25.3324256 |
| CoV274914 | ND | ND | ND | 28.901178 |
| CoV274915 | ND | ND | ND | 24.50090768 |
| CoV274916 | ND | ND | ND | 27.22745985 |
| CoV274917 | 27.07410327 | 25.386705 | 27.1454328 | 24.90575607 |
| CoV274918 | ND | ND | ND | 20.43756499 |
| CoV274919 | 27.2368955 | 26.2877203 | 28.3911897 | 29.31334367 |
| CoV274920 | ND | ND | ND | 29.18842679 |
| CoV274921 | ND | ND | ND | 26.02668508 |
| CoV274922 | ND | ND | ND | 28.3186006 |
| CoV274923 | ND | ND | ND | 24.74057186 |
| CoV274924 | ND | ND | ND | 22.99103722 |
| CoV274925 | 35.05929047 | 33.0690569 | 35.1592245 | 23.53473732 |
| CoV274926 | 26.10594794 | 24.6364944 | 26.8506228 | 24.51383932 |
| CoV274927 | ND | ND | ND | 24.60740076 |
| CoV274928 | ND | ND | ND | 26.4211567 |
| CoV274929 | ND | ND | ND | 23.42746668 |
| CoV274930 | 27.85107567 | 26.3845208 | 28.3670715 | 24.12029024 |
| CoV274931 | ND | ND | ND | 28.37135494 |
| CoV274932 | ND | ND | ND | 24.76150954 |
| CoV274933 | 29.40928102 | 28.0215352 | 29.7690104 | 22.05558397 |
| CoV274934 | ND | ND | ND | 35.84663926 |
| CoV274935 | ND | ND | ND | 20.73442636 |
| CoV274936 | 32.51673035 | 30.8944439 | 32.3712645 | 25.03331447 |
| CoV274937 | ND | ND | ND | 24.83489088 |
| CoV274938 | 38.55378759 | 35.762218 | 37.2817871 | 26.18522576 |
| CoV274939 | ND | ND | ND | 24.40827995 |
| CoV274940 | ND | ND | ND | 23.03160434 |
| CoV274941 | ND | ND | ND | 30.19420153 |
| CoV274942 | 31.67689377 | 29.6253727 | 31.2131717 | 27.34934312 |
| CoV274943 | ND | ND | ND | 30.24736383 |
| CoV274944 | ND | ND | ND | 26.56093959 |
| CoV274945 | ND | ND | ND | 30.90948224 |
| CoV274946 | ND | ND | ND | 31.66731105 |
| CoV274947 | ND | ND | ND | 25.07039946 |
| CoV274948 | 31.09698519 | 29.2389074 | 31.2439086 | 31.2941841 |
| CoV274949 | ND | ND | ND | 29.74684933 |
| CoV274950 | ND | ND | ND | 26.29739498 |
| CoV274951 | ND | ND | ND | 21.34513695 |
| CoV274952 | ND | ND | ND | 23.56897317 |
| CoV274953 | ND | ND | ND | 23.55791732 |
| CoV274954 | ND | ND | ND | 23.83094883 |
| CoV274955 | 27.89514918 | 26.6203825 | 27.7990095 | 24.14047041 |
| CoV274956 | ND | ND | ND | 25.88364166 |
| CoV274957 | 28.8016916 | 27.6238987 | 29.0376203 | 26.04204747 |
| CoV274958 | 34.28140616 | 33.1887783 | 33.1885669 | 27.10262934 |
| CoV274959 | 32.71935273 | 30.3318277 | 30.8543065 | 23.5666089 |
| CoV274960 | ND | ND | ND | 27.64201895 |
| CoV274961 | 30.02529596 | 28.7032334 | 30.0563281 | 26.63369912 |
| CoV274962 | 27.68751153 | 26.6592226 | 27.9067651 | 29.45265306 |
| CoV274963 | ND | ND | ND | 23.33361451 |
| CoV274964 | ND | ND | ND | 23.25749792 |
| CoV274965 | ND | ND | ND | 22.75498302 |
| CoV274966 | 31.41297649 | 30.4675857 | 31.3180624 | 23.13941092 |
| CoV274967 | 34.21245464 | 33.1179861 | 33.5684784 | 24.48007481 |
| CoV274968 | ND | ND | ND | 23.50798954 |
| CoV274969 | ND | ND | ND | 22.48747172 |
| CoV274970 | ND | ND | ND | 23.71025011 |
| CoV274971 | ND | ND | ND | 23.77164828 |
| CoV274972 | 34.83000961 | 33.4815086 | 33.9079545 | 27.71229665 |
| CoV274973 | ND | ND | ND | 22.85621527 |
| CoV274974 | ND | ND | ND | 24.50981933 |
| CoV274975 | 33.80135652 | 32.3319116 | 33.2172978 | 22.93741497 |
| CoV274976 | ND | ND | ND | 24.01251966 |
| CoV274977 | ND | ND | ND | 24.18210711 |
| CoV274978 | ND | ND | ND | 22.66671648 |
| CoV274979 | 35.13149335 | 32.66061163 | 33.1111602 | 23.4547327 |
| CoV274980 | ND | ND | ND | 23.4537466 |
| CoV274981 | 29.50245092 | 28.5260522 | 28.4835311 | 22.24834207 |
| CoV274982 | ND | ND | ND | 21.90841273 |
| CoV274983 | ND | ND | ND | 22.68776878 |
| CoV274984 | 32.48583553 | 31.3268056 | 32.3441375 | 23.11248401 |
| CoV274985 | ND | ND | ND | 21.8381511 |
| CoV274986 | ND | ND | ND | 21.65623227 |
| NTC_IC | ND | ND | ND | ND |
| NTC_PCR | ND | ND | ND | ND |
| POZ_PCR | 36.8758394 | 33.8054202 | 34.846188 | 32.40464394 |

### Example 5: Use of the isolated DNA for viral diagnostic.

In this experiment, the possibility of isolation and detection of DNA viruses, specifically an HPV virus, were studied. The isolation kit according to Example 1 was used.

The HPV16 positive and negative patient samples were selected and isolated. Isolation of DNA was performed using the Ribospin TM vRD (GeneAll) and by the RNA/DNA isolation kit according to Example 1. Anyplex II HPV HR Detection (Seegene) system was used form the detection.

Isolation and detection were performed according to the manufacturer's instructions. Tests were also performed with negative and positive controls; controls from the above commercial kit were used.

The results are shown in Table 3 below. It was shown that the isolation kit of the invention has comparable sensitivity for the detection of the viral DNA positivity and for the genomic DNA analysis, which is also demonstrated on the analysis of the housekeeping gene of the real patient samples.

**Table 3: HPV detection assay in patient samples which were isolated by the two different methods, Rbospin TM vRD and RNA/DNA isolation kit according the present invention.**

| **No of the sample** | **Isolation by RibospinTM vRD** | | **Isolation by RNA/DNA isolation kit** | |
|---|---|---|---|---|
| | Detection HPV DNA | House-keeping gene (DNA) | Detection HPV DNA | House-keeping gene (DNA) |
| 8384 | negative | ++ | negative | ++ |
| 8385 | negative | ++ | negative | ++ |
| 8386 | negative | ++ | negative | +++ |
| 8387 | negative | ++ | negative | ++ |
| 8388 | negative | ++ | negative | ++ |
| 8391 | negative | ++ | negative | ++ |
| 8392 | HPV31 (+) | ++ | HPV31 (+) | ++ |
| 8397 | negative | ++ | negative | ++ |
| 8398 | HPV31 (+) | ++ | HPV31 (+) | ++ |
| 8256 | negative | +++ | negative | +++ |
| Control1 | HPV45 (++) | ++ | HPV45 (++) | ++ |
| Control2 | HPV16 (++) | +++ | HPV16 (++) | ++ |
| Control3 | negative | ++ | negative | ++ |

## Claims

1. A kit for isolation of nucleic acid, which contains:
- an aqueous lysis buffer containing 0.05 to 0.07 M sodium citrate dihydrate, 10 to 20 µM 3,3',5,5'-tetrabromo-m-cresolsulfophthalein (bromocresol green) and 50 to 60% (w/v) guanidine thiocyanate, and having a pH of 6;
- an aqueous reducing solution containing 10 to 20% (w/v) tris(2-carboxyethyl)phosphine (TCEP);
- paramagnetic beads;
- a washing solution which is 0.5 to 2 mM citrate buffer in a 1:1 to 1:5 mixture of water and ethanol;
- a precipitating component which is ethanol;
- an elution solution which is 3 to 7 mM Tris buffer.

2. The kit according to claim 1, wherein the lysis buffer contains 0.06 M sodium citrate dihydrate, 14 µM 3,3',5,5'-tetrabromo-m-cresol sulfophthalein, and 56 to 57% (w/v) guanidine thiocyanate.

3. The kit according to claim 1 or 2, wherein the reducing solution contains 14 to 15% (w/v) tris(2-carboxyethyl)phosphine in water.

4. The kit according to any one of claims 1 to 3, wherein the washing solution contains 1 mM citrate buffer in a 1:1 to 1:5 mixture of water and ethanol, having pH 6.

5. The kit according to any one of claims 1 to 4, wherein the elution solution is 5 mM Tris buffer having pH 8.5.

6. The kit according to any one of claims 1 to 5, which further contains a nucleic acid carrier, preferably an RNA carrier based on linear polyacrylamide.

7. The kit according to claim 6, wherein the components: lysis buffer, reducing solution, paramagnetic beads, and precipitation component, and optionally nucleic acid carrier, are combined to form one mixed component of the kit.

8. The kit according to claim 7, wherein the lysis buffer, the reducing solution, the nucleic acid carrier and the precipitation component in the mixed component are mixed in volume ratios of 10³ : 5 to 10 : 5 to 10 : 10³ to 3x10³, more preferably in a volume ratio of 10³: 6: 7: 2×10³.

9. A method for isolation of nucleic acid from a sample, comprising the steps of
- providing a first mixture containing lysis buffer, reducing solution, paramagnetic beads, and precipitation solution, and optionally a nucleic acid carrier,
- mixing the said first mixture with the sample to form a second mixture,
- separating the paramagnetic beads from the liquid phase of the second mixture,
- mixing the paramagnetic beads with a washing solution and then separating the paramagnetic beads from the liquid phase, this step is optionally repeated,
- mixing the paramagnetic beads with an elution solution and separating the RNA-containing supernatant from the paramagnetic beads.

10. The method according to claim 9, wherein the steps of separating of the paramagnetic beads from the supernatant are preferably carried out by placing the mixture on a magnetic stand or on a magnetic pad, allowing the paramagnetic beads to settle by the action of the magnetic field.
